Europäisches Patentamt

European Patent Office

Office européen des brevets

(11) Publication number: **0 350 871**
**A2**

## (12) EUROPEAN PATENT APPLICATION

(21) Application number: **89112671.6**

(22) Date of filing: **11.07.89**

(51) Int. Cl.4: **A61F 13/00**

(30) Priority: **13.07.88 IL 87092**

(43) Date of publication of application:
**17.01.90 Bulletin 90/03**

(84) Designated Contracting States:
**DE ES FR GB IT NL**

(71) Applicant: **Itshki, Aviram**
**P.O. Box 1394**
**Rehovot 76110(IL)**

(72) Inventor: **Itshki, Aviram**
**P.O. Box 1394**
**Rehovot 76110(IL)**

(74) Representative: **Drost, Peter Dipl.-Ing.**
**Günderodestrasse 14a**
**D-8000 München 82(DE)**

(54) **Sanitary towel for rectal area.**

(57) The sanitary towel for rectal area according to
the invention consists in a hygienic bandage having
the form of a triangle with rounded edges. Said
bandage has three layers: The absorbing layer 1, the
non-transmitting layer 2, the adherence means as
layer 3 which may be coated with a protecting layer
4.

Fig.1

Fig.2

## SANITARY TOWEL FOR RECTAL AREA

The present invention relates to a Hygienic Bandage to be used at the rectal area (Os annulus).

There are known hygienic bandages. However, the known ones are those which are used by women for specific sanitary purposes. Said hygienic bandages are not adaptable to be used at the rectal area as they do.not have the proper form and size. However, very often bandages for the rectal area are required, e.g. by people suffering from haemorrhoids. Moreover, said bandages do not adhere to clothes and they transmit liquids, which is undesirable, should some medicament or the like have to be applied to the bandage.

It has therefore been desirable to devise a hygienic bandage utilisable at the rectal area, which overcomes said drawbacks, i.e. has a suitable size and form, can adhere to the clothes, and does not transmit liquids. Such bandage should be relatively cheap, easy to manufacture and be simple to be connected to the clothes, e.g. the underwear.

The present invention thus consists in a hygienic bandage (as hereinbefore defined), having the form of a triangle, the head thereof being broken off and the lower angles being rounded off, said bandages having 3 layers from the inwards to the outwards:

(a) an absorbing layer

(b) a layer which does not transmit liquid; and

(c) adherence means to the clothes.

The absorbent layer can be made of any suitable material, i.e. gauze, cotton, paper, and the like.

The non-transmitting layer is advantageously made from a suitable plastic material; rubber or the like.

The adherence means may be of any suitable means, such as plaster, gluing material and the like. If desired, it may consist of two parts, i.e. the adherence means proper and of some protective means, said protecting means are removed before use.

The hygienic bandage according to the present invention may be made of any adequate size and form and thus be adaptable to the body of the people who use it. It is advantageous worn in such a manner that the base of the triangle is pointin upwards and head downwards so that it adapts to the form of the body.

The bandage according to the present invention may be flat or have any other suitable form, i.e. be concave.

The hygienic bandage, according to the present invention, may be, if desired, provided with a medicament or with a sedative which is applied to the inner layer of the bandage, i.e. to the absorbing one.

The present invention will now be illustrated with regard to the accompanying drawings, without being restricted by same. In said drawings:

Fig. 1 shows the front view of the hygienic bandage according to the present invention in the open position; and

Fig. 2 shows a section across line II or Fig. 1.

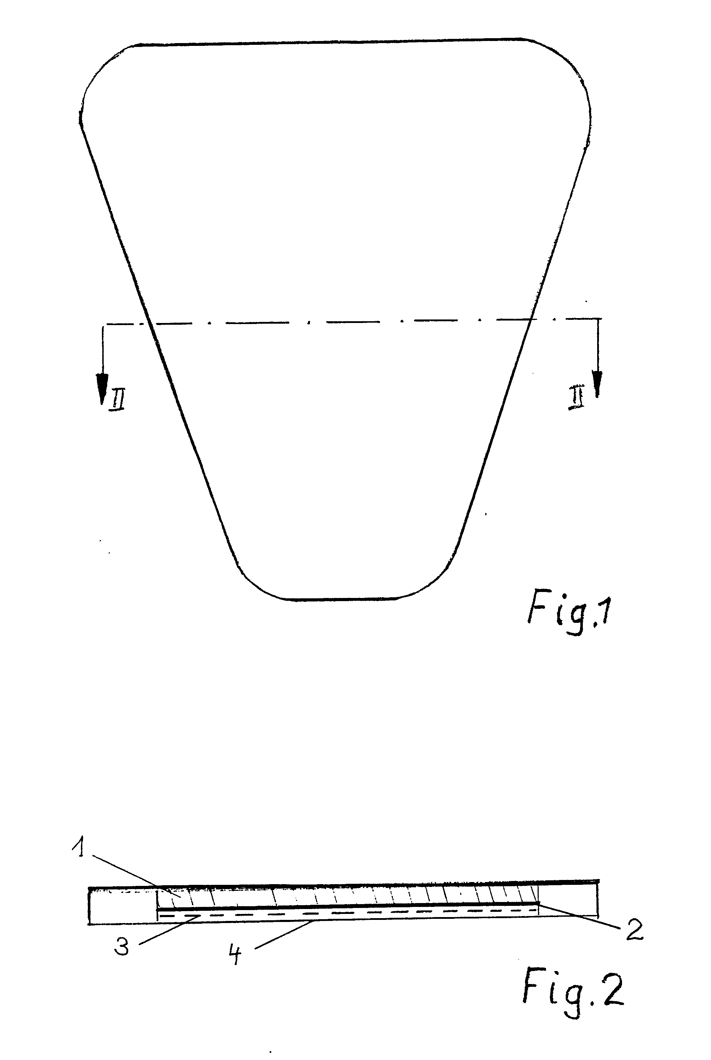

The layers indicated in Fig. 2 show absorbing layer 1, non-transmitting layer 2, adherence layer 3 and protecting layer 4.

## Claims

1. A hygienic bandage (as hereinbefore defined), having the form of a triangle, the head thereof being broken off and the lower angles being rounded off, said bandages having 3 layers from the inwards to the outwards:

(a) an absorbing layer

(b) a layer which does not transmit liquid; and

(c) adherence means to the clothes.

2. A hygienic bandage according to Claim 1, wherein the absorbent layer is made from a material selected from gauze, cotton and paper.

3. A hygienic bandage according to Claim 1 or 2, wherein the absorbing layer is provided with a medicament, a sedative or the like.

4. A hygienic bandage according to any of Claims 1 to 3, wherein non-transmitting layer is made of a suitable plastic material, rubber or the like.

5. A hygienic bandage according to any of Claims 1 to 4, wherein the adherence layer is made from a plaster, gluing material or the like.

6. A hygienic bandage according to any of Claims 1 to 5 being provided with protecting means to be removed before use.

7. A hygienic bandage according to any of Claims 1 to 6, being flat.

8. A hygienic bandage according to any of Claims 1 to 7, being concave.

9. A hygienic bandage, substantially as hereinbefore described with reference to the accompanying drawings.

Fig.1

Fig.2